# EUROPEAN PATENT APPLICATION

(11) **EP 1 396 265 A1**
(43) Date of publication of application: **10.03.2004**
(21) Application number: 03255222.6
(22) Date of filing: 22.08.2003
(51) Int. Cl.: A61K 31/137, A61K 31/663, A61K 31/675, A61K 47/48

(54) **Ion exchange resins for alleviation of upper gastrointestinal irritation**

(30) Priority: 04.09.2002 US 408113 P
(71) Applicant: ROHM AND HAAS COMPANY, Philadelphia, Pennsylvania 19106-2399 (US)
(72) Inventor: Will, Joanne Patricia, Easton, Pennsylvania 18042 (US); Hughes, Lyn, Harleysville, Pennsylvania 19438 (US)
(74) Representative: Kent, Venetia Katherine

(57) **Abstract**

The present invention is directed to a pharmaceutical composition for administering active ingredients that cause upper gastrointestinal tract irritation, such as polyphosphonates. Said dosage form comprises the irritating active ingredient and an ion exchange resin. Said dosage form protects the tissues of the buccal cavity, pharynx, esophagus, and the stomach from erosion and ulceration due to exposure of the irritating active ingredient to said tissues.

## Description

### BACKGROUND OF THE INVENTION

Active ingredients that cause upper gastrointestinal tract irritation present problems with patient compliance. A specific example of a class of active ingredients that have this problem are the polyphosphonic acids and their pharmaceutically-acceptable salts. They have been proposed for use in the treatment and prophylaxis of a number of pathological conditions which can affect humans or other mammals and involve calcium and phosphate metabolism. Such conditions may be divided into two broad categories:
1. Conditions which are characterized by anomalous mobilization of calcium and phosphate leading to general or specific bone loss or excessively high calcium levels in the fluids of the body. Such conditions are sometimes referred to herein as pathological hard tissue demineralizations.
2. Conditions which cause or result from deposition of calcium and phosphate anomalously in the body. These conditions are sometimes referred to herein as pathological calcifications.

The first category includes osteoporosis, a condition in which bone hard tissue is lost disproportionately to the development of new hard tissue. Marrow and bone spaces become larger, fibrous binding decreases, and compact bone becomes fragile. Osteoporosis can be subclassified as menopausal, senile, drug induced (e.g., adrenocorticoid, as can occur in steroid therapy), disease induced (e.g., arthritic and tumor). However, the manifestations are essentially the same. Another condition in the first category is Paget's disease (osteitis deformans). In this disease, dissolution of normal bone occurs which is then haphazardly replaced by soft, poorly mineralized tissue such that the bone becomes deformed from pressures of weight bearing, particularly in the tibia and femur. Hyperparathyroidism, hypercalcemia of malignancy, and osteolytic bone metastases are conditions also included in the first category.

The second category, involving-conditions manifested by anomalous calcium and phosphate deposition, includes myositis ossificans progressive, calcinosis universalis, and such afflictions as arthritis, neuritis, bursitis, tendonitis, and other inflammatory conditions which predispose involved tissue to deposition of calcium phosphates.

In particular polyphosphonates, like ethane-1-hydroxy-1,1-diphosphonic acid (EHDP), propane-3-amino-1-hydroxy-1,1-diphosphonic acid (APD), and dichloromethane diphosphonic acid have been the subject of considerable research efforts in this area. Paget's disease and heterotopic ossification are currently successfully treated with EHDP. The diphosphonates tend to inhibit the resorption of bone tissue, which is beneficial to patients suffering from excessive bone loss. However, EHDP, APD, and many other prior art diphosphonates have the propensity of inhibiting bone mineralization when administered at high dosage levels.

The compounds risedronate and alendronate are more biologically potent polyphosphonate compounds which can be administered at low dosage levels. These lower dosage levels have resulted in a wider margin of safety and cause little or no mineralization inhibition. It is believed that the decrease in the inhibition of bone mineralization which is exhibited by the low dosage levels occurs because mineralization inhibition is predominately a mass related physiochemical effect, whereas resorption inhibition results from a biological interaction with the cells. In addition, low dosage levels are also desirable to avoid the gastrointestinal discomfort, like nausea, diarrhea, and abdominal pains, which are sometimes associated with the oral administration of polyphosphonates.

Despite the low-dosage levels possible with risedronate and alendronate, the oral administration of these compounds sometimes result in patient complaints shortly after dosing. Said complaints are usually characterized by the patients as heartburn, esophageal burning, pain and/or difficulty upon swallowing, and/or pain existing behind and/or mid-sternum. It is believed that these complaints originate from esophagitis or esophageal irritation caused by the erosion, ulceration, or other like irritation of the epithelial and mucosal tissues of the upper gastrointestinal tract, generally the mouth through the esophagus, most generally the esophagus. It is hypothesized that said irritation results from the polyphosphonic acid active ingredient coming in direct contact with those epithelial and mucosal tissues, resulting in the topical irritation thereof.

Accordingly, it would be desirable to develop oral dosage forms of the polyphosphonic acid compounds which would prevent the release of polyphosphonic acid compounds in the area of said tissues. Said oral dosage forms would delay the beginning of the release of the polyphosphonic acid compound until some point in the small intestine or large intestine is reached and, thereby, provide protection to the tissues of the mouth, pharynx, and esophagus. The art has attempted to address these issues. For example, U.S. 6,096,342 discloses an enteric coated oral dosage form for risedronate comprising risedronate and excipients such as lactose monohydrate, microcrystalline cellulose, crospovidone, and magnesium stearate. U.S. 6,333,316 discloses oral alendronate dosage forms comprising a histamine H2 receptor blocker or a proton pump inhibitor and a bisphosphonate administered on a weekly, twice weekly, biweekly or twice monthly dosing schedule.

Now, Applicants have further contributed to the art of safely dosing active ingredients that cause esophageal irritation. Specifically, Applicants' invention relates to oral dosage forms of active ingredients that cause esophageal irritation comprising anion or cation exchange resins. Applicants dosage forms alleviate the problems associated with the oral administration of said compounds.

The following terms have the following meanings herein:

The term "active ingredient that causes upper gastrointestinal tract irritation", as used herein, includes but is not limited to the following active ingredients: polyphosphonic acids effecting bone metabolism such as alendronate and risedronate; antimuscarinics such as terodiline HCl and emepronium bromide and antibacterials such as doxycycline.

The term "gastrointestinal tract" as used herein relates to the alimentary canal, i.e., that musculomembranous tube about thirty feet in length, extending from the mouth to the anus. The term "upper gastrointestinal tract" as used herein means the buccal cavity, the pharynx, the esophagus, and the stomach. The term "lower gastrointestinal tract" as used herein means the small intestine, and the large intestine.

The term "buccal cavity" means the mouth or oral cavity and is lined with a mucous membrane which is continuous with the integument of the lips and with the mucous lining of the pharynx.

The term "pharynx" relates to the part of the upper gastrointestinal tract which is placed behind the nose, mouth, and larynx. It is a mucomembraneous tube about 4 inches in length and it is contiguous anteriorly with the mouth and posteriority with the esophagus and is composed of a mucous coat, a fibrous coat, and a muscular coat.

The term "esophagus" as used herein is a muscular canal about nine inches long extending from the pharynx to the stomach. The esophagus has three coats: an internal mucous coat surrounding the lumen, a middle areolar coat, and an external muscular coat.

The term "stomach" as used herein means that part of the gastrointestinal tract between the esophagus and the small intestine.

The term "small intestine" as used herein means that part of the lower gastrointestinal tract consisting of the duodenum, the jejunum, and the ileum, i.e., that portion of the intestinal tract just distal to the duodenal sphincter of the fundus of the stomach and proximal to the large intestine.

The term "large intestine" as used herein includes that part of the lower gastrointestinal tract just distal to the small intestine, beginning with the cecum, including the ascending colon, the transverse colon, the descending colon, the sigmoid colon, and the rectum.

The term "esophageal irritation" as used herein means irritation of any part of the upper gastrointestinal tract.

The term "pharmaceutical composition" means a dosage form comprised of a safe and effective amount of an active ingredient that causes upper gastrointestinal irritation and pharmaceutically-acceptable excipients.

The phrase "safe and effective amount," as used herein, means an amount of a compound or composition high enough to significantly positively modify the symptoms and/or condition to be treated, but low enough to avoid serious side effects (at a reasonable benefit/risk ratio), within the scope of sound medical judgment. The safe and effective amount of active ingredient for use in the method of the invention herein will vary with the particular condition being treated, the age and physical condition of the patient being treated, the severity of the condition, the duration of the treatment, the nature of the concurrent therapy, the particular active ingredient being employed, the particular pharmaceutically-acceptable excipients utilized, and like factors within the knowledge and expertise of the attending physician.

The term "pharmaceutically-acceptable excipients" as used herein includes any physiologically inert, pharmacologically inactive material known to one skilled in the art, which is compatible with the physical and chemical characteristics of the particular active ingredient selected for use. Pharmaceutically-acceptable excipients include, but are not limited to, polymers, resins, plasticizers, fillers, lubricants, solvents, co-solvents, buffer systems, surfactants, preservatives, sweetening agents, flavoring agents, pharmaceutical grade dyes or pigments, and viscosity agents. All or part of the pharmaceutically-acceptable excipients contained in the pharmaceutical compositions described herein is used to make the enteric-coating which is to be utilized in the novel oral dosage forms described herein.

The term "oral dosage form" as used herein means any pharmaceutical composition intended to be administered to the gastrointestinal tract of an individual via the mouth of said individual.

### SUMMARY OF THE INVENTION

The present invention relates to a pharmaceutical composition comprising:
(a) 5-75% by weight of an active ingredient that causes upper gastrointestinal tract irritation;
b) 25-95% by weight of an ion exchange resin.

### DETAILED DESCRIPTION OF THE INVENTION

The present invention relates to a pharmaceutical composition comprising:
(a) 5-75% by weight of an active ingredient that causes upper gastrointestinal tract irritation;
(b) 25-95% by weight of an ion exchange resin.

Active ingredients that cause esophageal irritation include, but are not limited to, polyphosphonic acids effecting bone metabolism such as alendronate and risedronate; antimuscarinics such as terodiline HCl and emepronium bromide and antibacterials such as doxycycline.

Polyphosphonic acid active ingredients effecting bone metabolism are particularly preferred in the practice of the present invention. Polyphosphonic acid active ingredients useful in the practice of the present invention include, but are not limited to, those described in U.S. 5,856,314; U.S. 5,853,759; U.S. 5,824,661; U.S. 5,770,586; U.S. 5,753,634; U.S. 5,731,299; U.S. 5,583,016; U.S. 5,574,024; U.S. 5,532,226; U.S. 5,527,940; U.S. 5,519,013; U.S. 5,508,273; U.S. 5,393,748; U.S. 5,393,746; U.S. 5,391,743; U.S. 5,334,586; U.S. 5,137,880; U.S. 5,104,863; U.S. 5,071,840; U.S. 4,939,131; U.S. 4,902,679; U.S. 4,868,164; U.S. 4,687,768; U.S. 4,922,007; U.S. 5,019,651; and U.S. 5,510,517.

Most preferred polyphosphonic acid active ingredients useful in the practice of the present invention are selected from the group consisting of :
alendronate - (4-amino-1-hydroxy-butylidene)bis-phosphonate;
cimadronate - ([(cycloheptylamino)methylene]bis-phosphonate;
clodronate - ((dichloromethylene)-bis-phosphonate;
EB-1053 - ([1-hydroxy-3-(1-pyrrolidinyl)-propylidene]bis-phosphonate;
etidronate - ((1-hydroxyethylidene)-bis-phophonate;
ibandronate - ([1-hydroxy-3-(methylpentylamino) propylidene]bis-phosphonate;
neridronate - ((6-amino-1-hydroxyhexylidene)bis-phosphonate;
olphadronate - ([3-(dimethylamino)-1-hydroxy-propylidene]bis-phosphonate;
pamidronate - ((3-amino-1-hydroxypropylidene)bis-phosphonate;
risedronate - ([1-hydroxy-2-(3-pyridinyl)-ethylidene]bis-phosphonate;
tiludronate - ([[(4-chlorophenyl)thio]methylene]bis-phosphonate
YH 529 - ([1-hydroxy-2-imidazo-(1,2a)pyridin-3-ylethylidene]bis-phosphonate; and
zolendronate - ([1-hydroxy-2-(1H-imidazol-1-yl) ethylidene]bis-phosphonate.

The active ingredients causing upper gastrointestinal tract irritation are used in the pharmaceutical compositions of the present invention at levels of 5-75 weight percent, preferably, 10-70 weight percent, and most preferably at 15-65 weight percent.

Ion exchange resins useful in the practice of the present invention are anion and cation exchange resins. Preferably, said resins are suitable for human ingestion.

Preferred anion exchange resins include, but are not limited to, styrenic strongly basic anion exchange resins with a quaternary amine functionality having a weight capacity of 0.1 to 15 meq/g, and styrenic weakly basic anion exchange resins with a primary, secondary, or tertiary amine functionality having a weight capacity of 0.1 to 8.5 meq/g, and acrylic or methacrylic strongly basic anion exchange resins with a quaternary amine functionality having a weight capacity of 0.1 to 12 meq/g, and acrylic or methacrylic weakly basic anion exchange resins with a primary, secondary, or tertiary amine functionality having a weight capacity of 0.1 to 12 meq/g, and allylic and vinylic weakly basic anion exchange resins with a primary, secondary, or tertiary amine functionality having a weight capacity of 0.1 to 24 meq/g, that are suitable for human and animal ingestion.

Most preferred anion exchange resins include, but are not limited to, styrenic anion exchange resins with quaternary amine functionality with weight capacity of 0.1 to 6 meq/g and acrylic anion exchange resins with tertiary amine functionality with weight capacity of 0.1 to 12 meq/g, that are suitable for human and animal ingestion.

Preferred cation exchange resins include, but are not limited to, styrenic strongly acidic cation exchange resins with sulfonic or phosphonic acid functionalities having a weight capacity of 0.1 to 8 meq/g; and styrenic weakly acidic cation exchange resins with carboxylic or phenolic acid functionalities having a weight capacity of 0.1 to 8.5 meq/g; and acrylic or methacrylic weakly acidic cation exchange resins with a carboxylic or phenolic acid functionality with a weight capacity of 0.1 to 14 meq/g, that are suitable for human and animal ingestion.

Most preferred cation exchange resins include, but are not limited to, styrenic weakly acidic cation exchange resin with a phenolic functionality with a weight capacity of 0.1 to 8.5 meq/g; and a styrenic strongly acidic cation exchange resin with a sulfonic acid functionality with weight capacity of 0.1 to 8 meq/g, or a methacrylic weakly acidic cation exchange resin with a carboxylic acid functionality with weight capacity of 0.1 to 12 meq/g.

Ion exchange resins useful in this invention have a moisture content between 0% and the water retention capacity of said resin.

Ion exchange resins useful in this invention are in powder or whole bead form.

Strongly acidic and weakly acidic cation exchange resins useful in the practice of this invention are in the acid form or salt form or partial salt form.

Strongly basic anion exchange resins useful in the practice of this invention are in the salt form.

The present invention can be prepared by forming an active ingredient that causes upper gastrointestinal tract irritation / ion exchange complex, also known as a resinate, and blending with suitable excipients.

Said ion exchange resins useful in the practice of the present invention are used at levels of 25-95 weight percent, preferably, 30-90 weight percent, and most preferably 35-85 weight percent.
Pharmaceutically-acceptable excipients include, but are not limited to, polymers, resins, plasticizers, fillers, lubricants, solvents, cosolvents, surfactants, preservatives, sweetener agents, flavoring agents, buffer systems, pharmaceutical-grade dyes or pigments, and viscosity agents.

Flavoring agents among those useful herein include those described in Remington's Pharmaceutical Sciences, 18^{th} Edition, Mack Publishing Company, 1990, pp. 1288-1300, incorporated by reference herein. Dyes or pigments among those useful herein include those described in Handbook of Pharmaceutical Excipients, pp. 81-90, 1986 by the American Pharmaceutical Association & the Pharmaceutical Society of Great Britain, incorporated by reference herein.

Preferred buffer systems include, but are not limited to, potassium acetate, boric, carbonic, phosphoric, succinic, malic, tartaric, citric, acetic, benzoic, lactic, glyceric, gluconic, glutaric, and glutamic. Particularly preferred are phosphoric, tartaric, citric, and potassium acetate.

Preferred surfactants include, but are not limited to, polyoxyethylene sorbitan fatty acid esters, polyoxyethylene monoalkyl ethers, sucrose monoesters and lanolin esters and ethers.

Preferred preservatives include, but are not limited to, phenol, alkyl esters of parahydroxylbenzoic acid, benzoic acid and the salts thereof, boric acid and the salts thereof, sorbic acid and the salts thereof, chlorbutanol, benzyl alcohol, thimerosal, phenylmercuric acetate and nitrate, nitromersol, benzalkonium chloride, cetylpyridinium chloride, methyl paraben, and propyl paraben. Particularly preferred are the salts of benzoic acid, cetylpyridinium chloride, methyl paraben, and propyl paraben.

Preferred sweeteners include, but are not limited to, sucrose, glucose, saccharin, and aspartame. Particularly preferred are sucrose and saccharin.

Preferred viscosity agents include, but are not limited to, methylcellulose, sodium carboxymethylcellulose, hydroxypropylmethylcellulose, carbomer, povidone, acacia, guar gum, xanthan gum, and tragacanth. Particularly preferred are methylcellulose, carbomer, xanthan gum, guar gum, povidone, and sodium carboxymethylcellulose.

Preferred fillers include, but are not limited to, lactose, sucrose, maltodextrin, and microcrystalline cellulose.

Preferred plasticizer include, but are not limited to, polyethylene glycol, propylene glycol, dibutyl phthalate, and castor oil, acetylated monoglycerides, and triacetin.

Preferred polymers include, but are not limited to, ethylcellulose, hydroxypropylmethylcellulose phthalate, and cellulose acetate phthalate, polyvinyl acetate phthalate, and Eudragit L-30-D.RTM., Eudragit L-100-55.RTM., and Eudragit S.RTM. 100, both manufactured by Rohm Pharma GmbH, Weiderstadt, West Germany, Cotteric, manufactured by Coloncon, Inc., West Point, PA.

Preferred lubricants include, but are not limited to, magnesium stearate, stearic acid, and talc.

The dosage forms of the present invention are prepared according to the following non-limiting examples:

### EXAMPLE 1 Preparation of risedronate resinate of an anion exchange resin

10g of risedronic acid is dissolved in 1 liter of water. 20g of cholestyramine USP is then added to 200mls of 1N sodium hydroxide in a separate container, and the slurry shaken for 2 hours. The slurry is then filtered and the resin rinsed with water. The resin is then added to the risedronic acid solution and shaken overnight. 5% hydrochloric acid is then added very slowly with stirring until the pH is in the range 8-10. This slurry is filtered and the resinate washed with water and dried for 6 hours at 60°C *in vacuo.* The risedronate resinate contains approximately 35 percent by weight of risedronate anion. 1g of the resinate contains the equivalent of 0.38g of risedronate sodium.

### EXAMPLE 2 Preparation of alendronate resinate of an anion exchange resin

15g of alendronic acid is dissolved in 1 liter of water. 25g of a weakly basic anion exchange resin with a weight capacity of approximately 10.1meq/g is then added to the alendronic acid solution and shaken overnight. This slurry is filtered and the resinate washed with water and dried for 6 hours at 60°C *in vacuo.* The alendronate resinate contains approximately 37.5 percent by weight of alendronate anion. 1g of the resinate contains the equivalent of 0.49g of monosodium alendronate trihydrate.

### EXAMPLE 3 Preparation of terodiline resinate of a cation exchange resin

2.4g of terodiline free base is dissolved in 350ml of 50 percent aqueous ethanol. 2.9g of a weakly acidic cation exchange resin powder in the hydrogen form with a weight capacity of approximately 10.6meq/g is then added to the solution and the slurry mixed overnight. The slurry is then filtered and the wetcake washed twice with 5g of aqueous ethanol each time. The wetcake is then dried for 4 hours at 60°C. The terolidine resinate contains approximately 45 percent by weight terolidine free base. 1g of the resinate contains the equivalent of 0.6g of terolidine hydrobromide.

## Claims

1. A pharmaceutical composition comprising:
(a) 5-75 percent by weight of an active ingredient that causes upper gastrointestinal tract irritation;
(b) 25-95 percent by weight of an ion exchange resin.

2. A pharmaceutical composition comprising:
(a) 5-75 percent by weight of an active ingredient that causes esophageal irritation;
(b) 25-95 percent by weight of an ion exchange resin.

3. A pharmaceutical composition according to Claim 2, wherein said active ingredient is a polyphosphonic acid.

4. A pharmaceutical composition according to Claim 3, wherein said polyphosphonic acid is risedronate.

5. A pharmaceutical composition comprising:
(a) 5-75 percent by weight of an active ingredient that causes esophageal irritation;
(b) 25-95 percent by weight of an anion exchange resin.

6. A pharmaceutical composition comprising:
(a) 5-75 percent by weight of an active ingredient that causes esophageal irritation;
(c) 25-95 percent by weight of a cation exchange resin.
